# EUROPÄISCHE PATENTSCHRIFT

(11) **EP 1 211 993 B1**
(45) Veröffentlichungstag und Bekanntmachung des Hinweises auf die Patenterteilung: **26.10.2005**
(21) Anmeldenummer: 00962501.3
(22) Anmeldetag: 14.09.2000
(51) Int. Cl.: A61B 17/80, A61B 17/72

(54) **FIXATIONSSYSTEM FÜR KNOCHEN**
FIXATION SYSTEM FOR BONES
SYSTEME DE FIXATION POUR OS

(30) Priorität: 14.09.1999 DE 19943924; 23.12.1999 DE 19962317
(43) Veröffentlichungstag der Anmeldung: 12.06.2002
(73) Patentinhaber: Wolter, Dietmar, Prof. Dr., D-21033 Hamburg (DE)
(72) Erfinder: Wolter, Dietmar, Prof. Dr., D-21033 Hamburg (DE)
(74) Vertreter: Siemons, Norbert
(86) Internationale Anmeldenummer: PCT/EP2000/009118
(87) Internationale Veröffentlichungsnummer: WO 2001/019268

(56) Entgegenhaltungen:
- DE-A- 4 343 117
- DE-A- 19 629 011
- DE-A- 19 858 889
- FR-A- 742 618
- US-A- 5 147 361
- US-A- 5 306 275
- US-A- 5 558 674
- US-A- 5 620 445

## Beschreibung

Die Erfindung bezieht sich auf ein Fixationssystem für Knochen mit einem Kraftträger mit Löchern und in die Löcher einsetzbaren Knochenschrauben.

Müssen Knochenbruchstücke miteinander verbunden werden, so stehen heute Platten-, Nagel- und Fixateursysteme zur Verfügung. Bisher wurde bei Platten- und Nagelsystemen die Stabilität dadurch erreicht, daß bei den Osteosyntheseplatten Knochenschrauben diese Platten fest an den Knochen heranzogen, um so durch den Anpreßdruck der Platte an den Knochen eine Stabilisierung der Knochenbruchstücke zu erreichen. Bei dem Marknagel, der im Markraum liegt, wird die Stabilität ebenfalls dadurch erhöht, daß Schrauben quer durch Knochen und Marknagel eingebracht werden. Diese Schrauben liegen zwar im Knochen mit einem Gewinde fest, die Durchquerung der Schraube durch den Nagel läßt jedoch kleinere Bewegungen zu.

Durch unterschiedliche technische Lösungen ist es gelungen, den Schraubenkopf mit der Platte fest zu verbinden bzw. eine feste Verbindung zwischen der Schraube und dem Marknagel selbst herzustellen. Hierzu wird auf die EP 0 201 024 B1, DE 43 43 117 A1, DE 196 29 011 A1 und die deutsche Patentanmeldung P 198 58 889.5 Bezug genommen. Gemäß DE 4343117 A1 und deutscher Patentanmeldung 198 58 889.8 ist es möglich, eine Knochenschraube unter verschiedenen Winkeln in einen Kraftträger einzuschrauben. Die Winkelveränderlichkeit bezieht sich auf eine Gewindeachse bzw. auf die Achse eines umlaufenden, umformbaren Vorsprunges, die senkrecht zum Verbindungsträger ausgerichtet ist.

Bei dieser neuen Generation von Implantaten kann man daher von inneren Fixateursystemen sprechen, da das Hauptmerkmal der äußeren Fixateure die Winkelstabilität zwischen Schraube und dem queren Kraftträger ist.

In der klinischen Anwendung zeigt sich bisher deutlich eine Überlegenheit dieser Fixateur intern-Systeme gegenüber herkömmlichen Platten- und Nagelsystemen.

Bei bekannten Fixationssystemen sind die Löcher senkrecht zum Kraftträger ausgerichtet.

Belastet der Patient entgegen ärztlichem Rat zu früh mit zu viel Körpergewicht, so kann es zur Verbiegung des Implantates bzw. zum Implantatbruch kommen.

Es ist beobachtet worden, daß es bei weichen Knochen und/oder einer hohen Belastung insbesondere auf Biegung zum Herausreißen der Schrauben aus dem Knochen kommen kann.

Die FR-A-742 618 offenbart schräg ausgerichtete Löcher in Verbindungsstücken für die Osteosynthese. Die Löcher weisen Gewinde auf, deren Gewindeachse exakt mit der Lochachse zusammenfällt. Zudem haben die Verbindungsstücke im Lochbereich eine Verdickung des Materials. Dies hat zur Folge, daß die Knochenschrauben nur exakt koaxial zur Lochachse eingesetzt werden können. Infolgedessen wird nur eine unidirektionale Stabilität erreicht und ist das Implantat aufwendig in der Herstellung.

Weitere Implantate mit schräg gerichteten Löchern und unidirektional einsetzbaren Knochenschrauben sind bekannt aus der US-A-5 306 275, US-A-5 147 361 und US-A-558 674.

Davon ausgehend liegt der Erfindung die Aufgabe zugrunde, ein Fixationssystem für Knochen zu schaffen, das das Einbringen von Knochenschrauben in den Knochen in einer wählbaren Winkelausrichtung unter besonders kleinen Winkeln zwischen Knochenschraube und Kraftträger ermöglicht.

Die Aufgabe wird durch ein Fixationssystem für Knochen mit den Merkmalen des Anspruches 1 und durch ein Fixationssystem für Knochen mit den Merkmalen des Anspruches 2 gelöst. Vorteilhafte Ausgestaltungen der Fixationssysteme sind in den Ansprüchen 2 bis 9 angegeben.

Der Erfindung liegt die Erkenntnis zugrunde, daß es je nach Knochenregion optimale Lagen der Knochenschrauben gibt, um die Verankerung des Kraftträgers, der insbesondere ein Implantat sein kann, im Knochen möglichst günstig zu gestalten. Dabei handelt es sich in erster Linie um Lagen, bei denen die Knochenschrauben eine Schrägausrichtung zum Kraftträger bzw. zum Abschnitt des Kraftträgers aufweisen würden, der im wesentlichen parallel zum Knochen bzw. zur Knochenregion verläuft, in dem bzw. der die Knochenschraube verankert werden soll. Deshalb kann das Fixationssystem für Knochen mit einem Kraftträger mit Löchern und in die Löcher einsetzbaren Knochenschrauben mindestens ein Loch aufweisen, das schräg zum Kraftträger ausgerichtet ist. Dies bedeutet, daß das Loch eine Lochachse aufweist, die eine Schrägausrichtung zumindest zu dem Abschnitt des Kraftträgers aufweist, durch den das Loch verläuft. Hierdurch kann eine in das Loch eingesetzte Knochenschraube in der zugeordneten Knochenregion eine optimale Lage erhalten. Zusätzlich oder statt dessen kann das Fixationssystem mindestens ein senkrecht zum Kraftträger ausgerichtetes Loch aufweisen, wobei durch besondere Maßnahmen, die unten erläutert werden, dennoch eine Schrägausrichtung einer Knochenschraube in diesem Loch zum Kraftträger ermöglicht wird. Besonders augenfällig sind die Vorteile der Erfindung in den gelenknahen Bereichen des Knochens. Hier ist der Knochen in der Regel verbreitert. Eine schräge Einbringung der Knochenschrauben zum Kraftträger führt hier zu einer günstigeren Fixation. Insgesamt wird eine bessere Lastübertragung und Stabilisierung der Knochenbruchstücke erreicht. Dies kann sich auch auf eine verminderte Belastung des Fixationssystems auswirken und dessen Verbiegung oder Bruch venneiden helfen.

Grundsätzlich ist es möglich, daß das Fixationssystem mindestens ein Loch aufweist, das schräg zum Kraftträger ausgerichtet ist und mindestens ein weiteres Loch, das in herkömmlicher Weise eine senkrechte Ausrichtung zum Kraftträger aufweist. Es können aber auch sämtliche Löcher eine Schrägausrichtung zum Kraftträger haben. Dabei können die Lochachsen der verschiedenen Löcher verschiedene Winkel zum Kraftträger aufweisen. Ferner können sämtliche Löcher senkrecht zum Kraftträger ausgerichtet sein.

Besonders vorteilhaft ist die winkelstabile Verbindung von Knochenschraube und Kraftträger.

Gemäß einer Lösung weist mindestens ein Loch mindestens ein durch Eindrehen einer Knochenschraube umformbares Element auf, welches etwa in einer schräg zur Lochachse des Loches ausgerichteten Ebene verläuft. Das umformbare Element kann insbesondere wie oben als Lippe, Grat, Kante oder Gewinde ausgeführt sein. Bei dem Loch kann es sich insbesondere um ein schräg zum Kraftträger ausgerichtetes Loch handeln. Dann ist es beispielsweise möglich, besonders kleine Winkel zwischen Knochenschraube und Kraftträger zu erreichen. Das Loch kann aber auch ein senkrecht zum Kraftträger ausgerichtetes Loch sein. Dann erlaubt die schräge Ausrichtung des umformbaren Elementes eine Fixierung einer Knochenschraube in einer Schrägausrichtung zum Kraftträger auch in einem solchen Loch. Dabei ist die Ausrichtbarkeit der Knochenschraube durch eine entsprechende Gestaltung des Loches sicherzustellen.

Gemäß einer weiteren Lösung weist mindestens ein Loch ein Gewinde zum Eindrehen einer Knochenschraube auf, dessen Gewindeachse schräg zur Lochachse ausgerichtet ist. Hierdurch ist es wiederum möglich, bei schräg zum Kraftträger ausgerichteten Löchern beispielsweise einen besonders kleinen Winkel der Knochenschraube zum Kraftträger zu erreichen. Bei einem senkrecht zum Kraftträger ausgerichteten Loch ist hingegen eine optimale schräge Lage der Knochenschraube im Knochen erreichbar.

Für eine winkelstabile Schraubverbindung kann sich ein umformbares Element oder ein Gewinde auch an der Knochenschraube befinden, und zwar zusätzlich zu der Anordnung im Loch gemäß einer der obigen Ausgestaltungen oder auch statt dessen.

Bei sämtlichen vorerwähnten winkelstabilen Verbindungen kann auch das umformbare Element bzw. das Gewinde in einem beispielsweise sich konisch oder sphärisch erweiternden Loch ausgebildet oder Abschnitt desselben sein und/oder an der Unterseite eines Schraubenkopfes der Knochenschraube, der in das Loch einzusetzen ist. Falls das Loch einen angrenzenden zylindrischen Abschnitt aufweist, kann dessen Durchmesser so bemessen sein, daß darin die Schrägausrichtung eines Schaftes der Knochenschraube möglich ist. Dies gilt auch für die folgenden Lösungen und Ausgestaltungen.

Soweit die winkelstabilen Verbindungen ein umformbares Element haben, ermöglichen diese ein Eindrehen der Knochenschraube in verschiedenen Winkelstellungen zum Kraftträger. Dies kann insbesondere für eine Feineinstellung der Lage der Knochenschraube im Knochen genutzt werden. Darauf wird unten noch näher eingegangen.

Einer Ausgestaltung liegt die überraschende Erkenntnis zugrunde, daß Knochenschrauben insbesondere dann ausreißgefährdet sind, wenn sie parallel zueinander in den Knochen eingebracht werden. Um dieses Heraustreten aus dem Knochen zu vermeiden, werden mindestens zwei Löcher nicht parallel zueinander in den Kraftträger eingebracht, sondern schräg zueinander geneigt. Dafür kann gegenüber herkömmlichen Kraftträgern, bei denen die Löcher in einem Winkel von 90° zum Kraftträger (bzw. zu einer Zentralebene bzw. einer Auflageebene desselben am Knochen) eingebracht sind, mindestens ein Loch schräg zum Kraftträger geneigt sein. Vorzugsweise können zwei oder mehrere Löcher entsprechend zueinander geneigt im Kraftträger angeordnet sein. Bevorzugt wird dabei, daß Löcher, die auf verschiedenen Seiten einer Bruch- oder Instabilitätszone eines Knochens anzuordnen sind, in verschiedenen Richtungen zueinander geneigt im Kraftträger angeordnet sind. Durch die Verspreizung der Knochenschrauben im Knochengewebe wird eine bessere Lastübertragung ermöglicht.

Da der Knochen in der Regel gebogene Oberflächen aufweist und dieses insbesondere im gelenknahen Bereich der Fall ist, besteht die Notwendigkeit, daß insbesondere Plattensysteme dieser Knochenbiegung angepaßt werden. Dieser Vorgang erfolgt in der Regel durch entsprechende Biegewerkzeuge während der Operation. Dabei kann auch die Ausrichtung von Plattenlöchern entsprechend der Anmodellierung verändert werden. Findet sich eine deutliche Knochenoberflächenbiegung, wie im gelenknahen Bereich, so kann die schräge Anlegung des Schraubenloches das Erreichen einer optimalen Schraubenlage im Knochen weiter erleichtern. Dies kann bei der Ausrichtung von Löchern in der Platte von vornherein berücksichtigt werden, so daß nach dem Anmodellieren eine gewünschte schräge Ausrichtung mindestens zweier Löcher in der Platte erreicht wird.

Die Knochenschrauben sind unter verschiedenen Winkeln in die Löcher des Kraftträgers einsetzbar und in diesem fixierbar. Dabei können Kraftträger bzw. Schrauben gemäß den eingangs erwähnten Patentanmeldungen ausgestaltet sein, insbesondere gemäß DE 43 43 117 A1, DE 196 29 011 A1 oder P 198 58 889.5. Der Fixierung der Schrauben unter unterschiedlichen Winkeln im Kraftträger kann insbesondere ein umformbares Element dienen, das im Loch und/oder an der Schraube angeordnet ist und das ein Grat, eine Lippe, eine Kante oder ein Gewinde sein kann. Beim Eindrehen der Knochenschraube in das Loch unter Umformung des Materials ist es möglich, unterschiedliche Winkelausrichtungen der Knochenschraube im Kraftträger zu erreichen. Dabei ist beispielsweise eine Ausrichtbarkeit in einem Winkelbereich von 10° bis 15° zur Lochachse erreichbar. Auch höhere Winkelgrade sind jedoch unter erhöhtem Aufwand für die Materialumformung erzielbar. Eine schon vorgegebene, optimierte Richtung des Loches oder des umzuformenden Elementes kann es dem Operateur erleichtern, die Schraube in die optimale Position zu bringen, ohne viel Material im Knochenloch umzuformen.

Falls mindestens zwei Löcher im Kraftträger schräg zueinander geneigt sind, ist es möglich, mindestens zwei Knochenschrauben von vornherein zueinander geneigt in den Kraftträger einzubringen, ohne den durch die Fixierbarkeit unter verschiedenen Winkeln gegebenen Spielraum zu verbrauchen. Hierdurch werden die Möglichkeiten, durch Schräglage eine Verspreizung der Schrauben im Knochen zu erreichen, erheblich verbessert.

Bei beiden Lösungen kann das Fixationssystem insbesondere eine Knochenplatte, ein Knochennagel oder ein Fixateur sein.

Durch die schräge Ausrichtung der Löcher bzw. des umformbaren Elementes bzw. des Gewindes bezüglich des Kraftträgers läßt sich insbesondere ein Ausreißen bzw. eine Beschädigung des Implantates bei Überbeanspruchung vermeiden.

Die Erfindung wird nachfolgend anhand der anliegenden Zeichnungen von Ausführungsbeispielen näher erläutert. In den Zeichnungen zeigen:
- Fig. 1: eine Knochenplatte an einem Tibia-Knochen in Gelenknähe in der Draufsicht;
- Fig. 2: eine Knochenplatte einem senkrechten und zwei schräg ausgerichteten Löchern am Mittelbereich eines Röhrenknochens im Längsschnitt;
- Fig. 3: eine gebogene Knochenplatte an einem Tibia-Knochen in Gelenknähe in einem teilweisen Längsschnitt;
- Fig. 4: eine Knochenplatte mit einem senkrecht ausgerichteten Loch und umformbarem Element in einer schräg ausgerichteten Ebene in einem perspektivischen Ausschnitt;
- Fig. 5: eine Knochenplatte mit einem schräg ausgerichteten Loch und vorgeformtem Gewinde in einem perspektivischen Teilausschnitt.

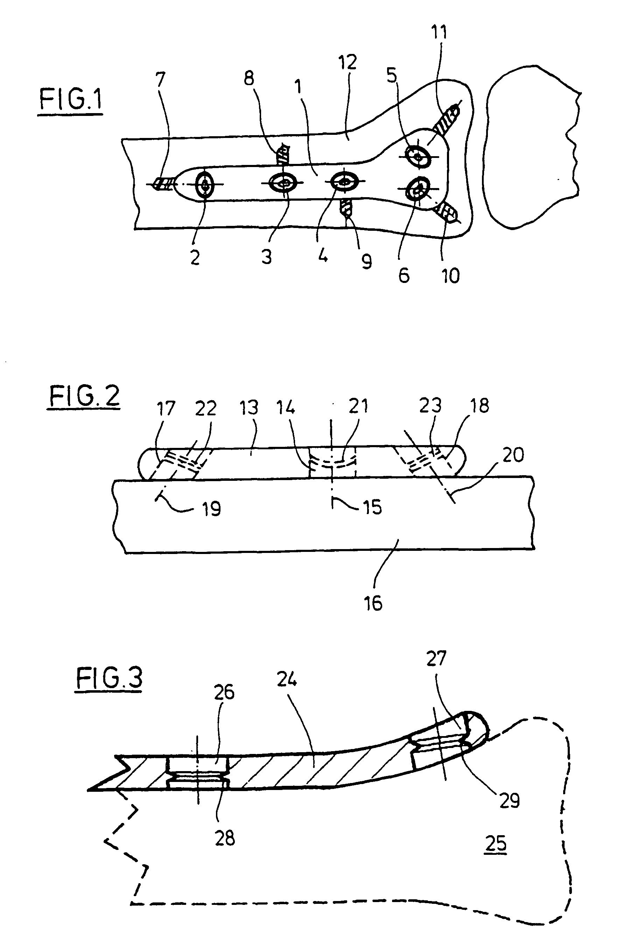

Die Figuren 1 bis 3 zeigen keine Ausführungsbeispiele der Erfindung, wohl aber Merkmale der beanspruchten Erfindung.

Gemäß Fig. 1 hat eine im wesentlichen T-förmige Knochenplatte 1 in dem länglichen Abschnitt drei Löcher 2, 3, 4 und einem kurzen, quergerichteten

Abschnitt zwei weitere Löcher 5, 6. Die Löcher 2 bis 6 haben jeweils eine Lochachse, die schräg zu dem Abschnitt der Knochenplatte 1 angeordnet ist, in dem sich das jeweilige Loch 2 bis 6 befindet. Bei einer gänzlich ebenen Knochenplatte 1 hat jedes Loch 2 bis 6 eine bestimmte Schrägausrichtung zu der Ebene durch die gesamte Knochenplatte 1.

In die Löcher 2 bis 6 sind Knochenschrauben 7, 8, 9, 10, 11 eingesetzt, deren Ausrichtung den Ausrichtungen der Löcher 2 bis 6 entspricht. Damit sind die Knochenschrauben 7 bis 1 optimal auf die Bereiche eines Tibia-Knochens 12 ausgerichtet, in die sie jeweils einzudrehen sind.

Die Knochenschrauben 7 bis 11 greifen mit ihren Schraubenköpfen in die Löcher 2 bis 6 ein.

Fig. 2 zeigt eine Knochenplatte 13, bei der das mittlere Loch 14 mit seiner Lochachse 15 konventionell senkrecht zur Knochenplatte 13 ausgerichtet ist. Infolgedessen wird eine in das Loch 14 eingedrehte Knochenschraube grundsätzlich senkrecht in einen darunter befindlichen Röhrenknochen 16 eingedreht.

Die beiden äußeren Löcher 17, 18 der Knochenplatte 13 sind jedoch mit ihren Achsen 19, 20 spitzwinklig zur Knochenplatte 13 ausgerichtet. Infolgedessen wird eine Verspreizung von in die beiden äußeren Löcher 17, 18 einzudrehenden Knochenschrauben in dem angrenzenden Knochen 16 erreicht und damit eine sichere Befestigung der Knochenplatte 13.

Zudem weisen sämtliche Löcher 14, 17, 18 ein Element 21, 22, 23 zur winkelstabilen Festlegung einer Knochenschraube auf, das als umformbarer Grat oder Lippe oder Kante oder als vorgeformtes Gewinde (umformbar oder auch nicht) ausgeführt sein kann. Im Beispiel ist das Element 21, 22, 23 zur winkelstabilen Festlegung in einer senkrecht zur Lochachse 15, 19, 20 ausgerichtete Ebene angeordnet, sofern es sich bei dem Element 21, 22, 23 um einen Grat, eine Lippe oder eine Kante handelt. Sofern es sich bei dem Element 21, 22, 23 um ein Gewinde handelt, fällt dessen Achse mit der Lochachse 15, 19, 20 zusammen.

Bei einer umformbaren Ausführung des Elementes 21, 22, 23 zur winkelstabilen Festlegung der Knochenschrauben besteht zudem die Möglichkeit, die Knochenschrauben unter unterschiedlichen Ausrichtungen bezüglich der Lochachsen 15, 19, 20 in den Löchern 14, 17, 18 zu fixieren. Dafür sind die Knochenschrauben mit ihren Schraubenköpfen in die Löcher 14, 17, 18 eingesetzt, wobei Gewinde an den Unterseiten der Schraubenköpfe mit den umformbaren Elementen 21, 22, 23 zusammenwirken.

Gemäß Fig. 3 ist eine Knochenplatte 24 durch Verbiegen so verformt, daß sie gut an den Gelenksbereich eines Tibia-Knochens 25 paßt. Sie weist Löcher 26, 27 auf. Auf dem abgeschnittenen Teil der Knochenplatte 24 können weitere Löcher vorhanden sein. Die Achse des Loches 26 ist senkrecht zur Knochenplatte 24 ausgerichtet. Die Achse des Loches 27 ist von vornherein zur Knochenplatte 24 bzw. deren Auflagefläche auf dem Knochen 25 geneigt. Dabei ist die Neigung der Achse des Loches 27 so geplant, daß nach dem Anmodellieren der Knochenplatte 24 an den Knochen 25 eine Schrägausrichtung der Achsen der Löcher 26, 27 zueinander vorhanden ist. Dies führt zu einer Verspreizung eingedrehter Schrauben im Knochen 25, die einem Ausreißen der Knochenplatte 24 aus dem Knochen 25 entgegenwirkt.

Gemäß Fig. 3 sind die Löcher 26, 27 der Knochenplatte 24 an ihrem Innenumfang mit einem umlaufenden Grat 28, 29 versehen. In diesen Grat 28, 29 kann eine Knochenschraube mit einem Gewinde an der Unterseite ihres Kopfes in verschiedenen Winkelstellungen eingedreht werden, wobei eine Umformung des Grates 28, 29 eintritt, je nachdem, in welchem Winkel zur Achse des Loches 26, 27 die Knochenschraube eingedreht wird. Zudem wird bei der Umformung des Grates bzw. des Gewindes der Schraube eine Sicherung der Schraube in der Eindrehstellung des Knochens bewirkt. Durch die vorgeplante Schräglage der Achsen der Löcher 26, 27 zueinander ist eine geneigte Ausrichtung der Knochenschrauben zueinander möglich, ohne den für die Fixierbarkeit unter verschiedenem Winkel in der Knochenplatte 24 gegebenen Spielraum zu verbrauchen. Somit wird zugleich eine Verspreizung und eine optimale Ausrichtbarkeit der Knochenschrauben in einer individuell optimierbaren Winkellage in ihren Löchern 26, 27 erreicht.

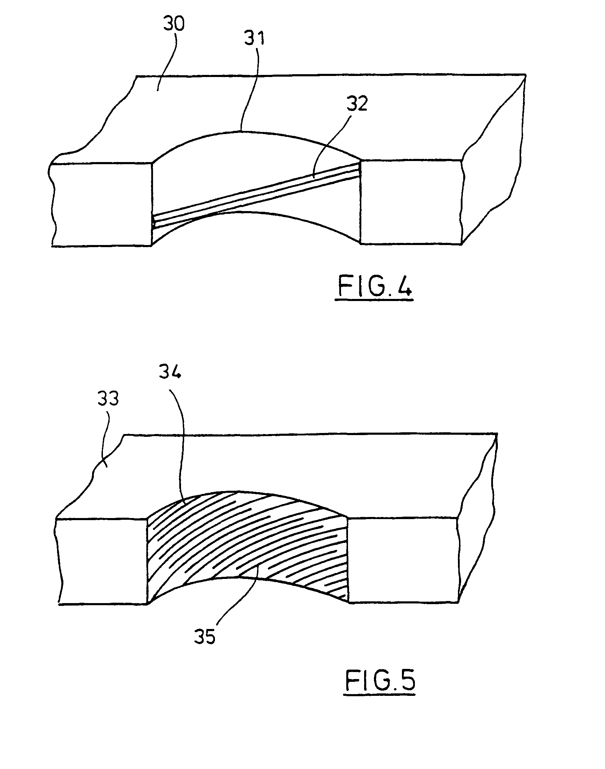

Gemäß Fig. 4 hat zwar eine Knochenplatte 30 ein in herkömmlicher Weise senkrecht zu dieser ausgerichtetes Loch 31. In dem Loch 31 befindet sich jedoch mindestens ein Element 32 zum winkelstabilen Fixieren einer Knochenschraube, das eine Schrägausrichtung zur Lochachse des Loches 31 hat. Das Element 32 kann insbesondere ein umformbarer Grat, Lippe oder Kante sein, wobei es dann in einer Ebene angeordnet ist, die schräg zur Lochachse geneigt ist. Es kann sich bei dem Element 32 aber auch um ein vorgeformtes Gewinde handeln, dessen Gewindeachse zur Lochachse geneigt ist. In beiden Fällen ermöglicht das Element eine Verankerung einer Schrauben in einer vorgegebenen Schrägausrichtung zur Knochenplatte 30, wobei durch die umformbare Ausführung des Elementes 32 eine gewisse zusätzliche Variation der Winkelstellungen möglich ist.

Gemäß Fig. 5 hat eine Knochenplatte 33 ein von vornherein schräg zu dieser ausgerichtetes Loch 34, das mit einem vorgeformten Gewinde 35 ausgestattet ist. Eine Knochenschraube ist in das Loch 34 in einer vorgegebenen Ausrichtung eindrehbar, die schräg zur Knochenplatte 33 ausgerichtet ist. Durch die umformbare Ausführung des Gewindes 35 ist eine gewisse Winkelausrichtbarkeit gegeben.

Auch bei den Ausführungen der Fig. 4 und 5 ist bevorzugt die Knochenschraube mit dem Schraubenkopf in dem jeweiligen Loch 31, 34 verankerbar. Der Schraubenkopf kann sich hierzu nach oben, d.h. zu der vom Knochen abgewandten Seite der Knochenplatte 30, 33 hin verjüngen.

## Patentansprüche

1. Fixationssystem für Knochen mit einem Kraftträger (1, 13, 24, 30) mit Löchem (2 bis 6; 14, 17, 18; 26, 27; 31) und in die Löcher einsetzbaren Knochenschrauben (7 bis 11), wobei mindestens ein Loch (31) mindestens ein durch Eindrehen einer Knochenschaube umformbares Element (32) ausgewählt aus Grat, Lippe oder Kante aufweist, **dadurch gekennzeichnet, daß** das umfombare Element (32) in einer schräg zur Lochachse des Loches ausgerichteten Ebene verläuft.

2. Fixationssystem für Knochen mit einem Kraftträger (1, 13, 24 , 33) mit Löchern (2 bis 6; 14, 17, 18; 26, 27; 34) und in die Löcher einsetzbaren Knochenschrauben (7 bis 11), wobei mindestens ein Loch (34) ein umformbares Gewinde (35) zum Eindrehen einer Knochenschraube aufweist, **dadurch gekennzeichnet, daß** die Gewindeachse des umformbaren Gewindes (35) schräg zur Lochachse ausgerichtet ist.

3. Fixationssystems nach Anspruch 1 oder 2, **dadurch gekennzeichnet, daß** mindestens ein Loch (31) senkrecht zum Kraftträger (30) ausgerichtet ist.

4. Fixationssystem nach einem der Ansprüche 1 bis 3, **dadurch gekennzeichnet, daß** mindestens ein Loch (2 bis 6; 17, 18; 27) schräg zum Kraftträger (1, 13, 24) ausgerichtet ist.

5. Fixationssystem nach einem der Ansprüche 1 bis 4, **dadurch gekennzeichnet, daß** mindestens zwei Löcher (17, 18; 26, 27) schräg zueinander geneigt sind.

6. Fixationssystem nach Anspruch 5, **dadurch gekennzeichnet, daß** die Achsen der beiden Löcher (17, 18; 26, 27) auf der dem Knochen (16, 25) zuzuwendenden Seite des Kraftträgers (13, 24) divergieren.

7. Fixationssystem nach einem der Ansprüche 1 bis 5, **dadurch gekennzeichnet, daß** der Kraftträger (24) entsprechend der Oberfläche eines Bereichs eines Knochens (25) geformt oder an eine solche Oberfläche anmodellierbar ist.

8. Fixationssystem nach einem der Ansprüche 1 bis 7, **dadurch gekennzeichnet, daß** ein umformbares Element (21, 22, 23; 28, 29; 32) ausgewählt aus Grat, Lippe oder Kante oder ein umformbarer Abschnitt mindestens eines Loches (14, 17, 18; 26, 27; 31) des Kraftträgers (13, 24, 30) und ein Schraubenkopf und/oder ein anderer Abschnitt der Knochenschraube Materialien mit unterschiedlichen Härtegraden aufweisen.

9. Fixationssystem nach einem der Ansprüche 1 bis 8, bei dem der Kraftträger (1, 13, 24, 30, 33) eine Knochenplatte, ein Knochennagel oder ein Fixateur ist.

## Claims

1. A fixation system for bones with a force support (1, 13, 24, 30) having holes (2 to 6; 14, 17, 18; 26, 27; 31) and bone screws (7 to 11) adapted to be inserted into the holes, in which at least one hole (31) has at least one element (32) deformable by turning in a bone screw which is selected from a ridge, lip or edge, **characterized in that** the deformable element (32) extends in a plane oriented obliquely to the hole axis of the hole.

2. A fixation system for bones with a force support (1, 13, 24, 33) having holes (2 to 6; 14, 17, 18; 26, 27; 34) and bone screws (7 to 11) adapted to be inserted into the holes, in which at least one hole (34) has a deformable thread (35) for turning in a bone screw, **characterized in that** the thread axis of which is oriented obliquely to the hole axis of the hole.

3. The fixation system according to claim 1 or 2, **characterized in that** at least one hole (31) is oriented perpendicularly to the force support (30).

4. The fixation system according to any one of claims 1 to 3, **characterized in that** at least one hole (2 to 6; 17, 18; 27) is oriented obliquely to the force support (1,13,24).

5. The fixation system according to any one of claims 1 to 4, **characterized in that** at least two holes (17, 18; 26, 27) are oriented obliquely towards each other.

6. The fixation system according to claim 5, **characterized in that** the axes of the two holes (17, 18; 26, 27) diverge on the side of the force support (16, 25) which is to face the bone (16, 25).

7. The fixation system according to any one of claims 1 to 5, **characterized in that** the force support (24) is formed according to the surface of an area of a bone (25) or is conformable to such a surface.

8. The fixation system according to any one of claims 1 to 7, **characterized in that** a deformable element (21, 22 23; 28, 29; 32) selected from a ridge, lip or edge or a deformable portion of at least one hole (14, 17, 18; 26, 27; 31) of the force support (13, 24, 30) and a screw head and/or another portion of the bone screw exhibit materials of different degrees of hardness.

9. The fixation system according to any one of claims 1 to 8 wherein the force support (1, 13, 24, 30, 33) is a bone plate, a bone nail or a fixateur.

## Revendications

1. Système de fixation d'os avec un support de forces (1, 13, 24, 30) comportant des trous (2 à 6 ; 14, 17, 18 ; 26, 27 ; 31) et des vis à os (7 à 11), insérables dans les trous, dans lequel au moins un trou (31) présente au moins un élément (32) déformable par vissage d'une vis à os, choisi sous forme de nervure, de lèvre ou d'arête, l'élément déformable (32) se situant dans un plan orienté de façon inclinée par rapport à l'axe de trou du trou.

2. Système de fixation d'os avec un support de forces (1, 13, 24, 33) comportant des trous (2 à 6 ; 14, 17, 18 ; 26, 27 ; 34) et des vis à os (7 à 11), insérables dans les trous, dans lequel au moins un trou (34) présente un filetage déformable (35) pour le vissage d'une vis à os, l'axe de filetage du filetage déformable (35) étant orienté de façon inclinée par rapport à l'axe du trou.

3. Système de fixation selon la revendication 1 ou 2, **caractérisé en ce qu'**au moins un trou (31) est orienté perpendiculairement au support de forces (30).

4. Système de fixation selon l'une des revendications 1 à 3, **caractérisé en ce qu'**au moins un trou (2 à 6 ; 17, 18 ; 27) est orienté de façon inclinée par rapport au support de forces (1, 13, 24).

5. Système de fixation selon l'une des revendications 1 à 4, **caractérisé en ce qu'**au moins deux trous (17, 18 ; 26, 27) sont penchés de façon inclinée l'un par rapport à l'autre.

6. Système de fixation selon la revendication 5, **caractérisé en ce que** les axes des deux trous (17, 18 ; 26, 27) divergent sur la face du support de forces (13, 24) dirigée vers l'os (16, 25).

7. Système de fixation selon l'une quelconque des revendications 1 à 5, **caractérisé en ce que** le support de forces (24) peut être formé en fonction de la surface d'une région d'un os (25) ou modelé sur une surface de ce type.

8. Système de fixation selon l'une quelconque des revendications 1 à 7, **caractérisé en ce qu'**un élément déformable (21, 22, 23 ; 28, 29 ; 32) choisi sous forme de nervure, de lèvre ou d'arête ou un segment déformable au moins d'un trou (14, 17, 18 ; 26, 27 ; 31) du support de forces (13, 24, 30) et une tête de vis et/ou un autre segment de la vis à os présentent des matériaux ayant différents degrés de dureté.

9. Système de fixation selon l'une quelconque des revendications 1 à 8, dans lequel le support de forces (1, 13, 24, 30, 33) est une plaque à os, un clou à os ou un fixateur.
